# EUROPEAN PATENT APPLICATION

(11) **EP 4 410 267 A1**
(43) Date of publication of application: **07.08.2024**
(21) Application number: 22874863.8
(22) Date of filing: 26.09.2022
(51) Int. Cl.: A61K 8/24, A61K 8/21, A61K 8/25, A61K 8/19, A61K 8/20, A61K 8/02, A61Q 11/00, A61P 1/02

(54) **DENTAL GEL HAVING REMINERALIZING ABILITY, DENTAL PATCH, AND METHOD FOR PREPARING DENTAL GEL**

(30) Priority: 28.09.2021 CN 202111143027
(71) Applicant: Central South University, Changsha, Hunan 410083 (CN)
(72) Inventor: CHEN, Xiaojing, Changsha, Hunan 410083 (CN)
(74) Representative: Vossius & Partner Patentanwälte Rechtsanwälte mbB
(86) International application number: PCT/CN2022/121473
(87) International publication number: WO 2023/051478

(57) **Abstract**

The present application discloses a tooth gel and a tooth strip with remineralization capability, and a preparation method of the tooth gel. The tooth gel includes the following raw material components by mass: 1-35% of active ingredients, 20-60% of thickening agent, 20-70% of solvent, 5-25% of solid dispersant, 0.1-0.8% of essence, 0.1-1.0% of sweetener and 0.1-1.0% of pH regulator; the raw materials for preparing the active ingredients include calcium phosphate and/or bioactive glass; and the tooth strip includes a three-layer structure: an anti-sticking liner, a dental gel layer and a peel-off backing layer, where the anti-sticking liner is adhered to one surface of the dental gel layer, the other surface of the dental gel layer is adhered to the a peel-off backing layer, and the dental gel layer is a tooth gel. The products of the present application are convenient to carry and use, have the functions of desensitization, remineralization, antibiosis, caries prevention, whitening and the like, have important function and significance for preventing and treating oral diseases, and have little or no irritation to oral soft tissues.

## Description

The present application claims priority to Chinese Patent Application No. 202111143027.2, entitled with "TOOTH GEL AND TOOTH STRIP WITH REMINERALIZATION CAPABILITY AND PREPARATION METHOD OF TOOTH GEL", filed with China National Intellectual Property Administration on September 28, 2021. The disclosure of the aforementioned application is hereby incorporated by reference in its entirety.

### TECHNICAL FIELD

The present application belongs to the technical field of oral health and biomimetic biomaterial engineering technology (biomineralization), and particularly relates to a tooth gel and tooth strip with remineralization capability and a preparation method of the tooth gel.

### BACKGROUND

Oral health is an important component of general health. However, at present, dental caries, periodontal disease and dentin hypersensitivity are still the most common oral diseases in the world, which seriously affects people's quality of life. The degree of these oral diseases develops progressively over time, and the remineralization of dental tissue is expected to block the development of these diseases and repair damaged teeth.

In addition, with the improvement of people's living standards, people's awareness of oral aesthetics is becoming stronger and stronger, and more and more attention is paid to dental cosmetology. Tooth whitening is one of the most popular dental cosmetic ways at present, which is mainly achieved by chemical bleaching and cover-whitening. Chemical bleaching mainly includes cold-light whitening, denture whitening, tooth strip whitening, gargle whitening, whitening by sealing drug in teeth and so on.

Among them, denture whitening and tooth strip whitening are the most widely used family whitening manners at present. Tooth strip whitening is more convenient and more common, and its price is cheaper than that of denture whitening.

The construction of whitening tooth strip typically includes: a plastic film layer, a gel layer, and a peel-off backing layer. The gel layer contains a substance with tooth whitening efficacy which is closely connected with the plastic film layer. When in use, the peel-off backing layer is torn off, a surface with the gel layer is adhered to the surface of teeth, and the tooth strip is adjusted by gently pressing it with a finger to be completely adhered to the surface of the teeth. After use, the tooth strip is taken out from the teeth and discarded. Whitening tooth strip is favored by consumers because of its advantages of convenience for carrying, ease of use, low price, etc. For example, according to Chinese patent application CN202011233081.1, a soluble oral whitening bio-membrane tooth strip includes the following components based on parts by mass: 50-75 parts of film forming agent, 1-5 parts of phthalimido-peroxycaproic acid, 1-3 parts of butylated hydroxytoluene, 1-3 parts of phosphoric acid and 5-15 parts of emulsifier; the tooth strip adopts phthalimido-peroxycaproic acid as oxidant, which has excellent whitening effect; the film carrier of the tooth strip is made of polyvinylpyrrolidone and hydroxypropyl cellulose, which can be dissolved in the oral cavity and is convenient to use and environment-friendly. As another example, Chinese patent application CN201710548647.1 discloses a whitening tooth strip containing silver-loaded activated carbon, including a three-layer structure, where a first layer is a soft plastic film, a second layer is a whitening bacteriostatic gel layer and a third layer is a peel-off backing layer; when in use, the soft plastic film together with the whitening bacteriostatic gel layer are torn off from the peel-off backing layer and then adhered to the tooth surface; the whitening bacteriostatic gel is composed of silver-loaded activated carbon and stain remover; and the product is safe, stable and non-irritating, has a whitening effect equivalent to that of a commercially available 6% HP whitening tooth strip, and is effective in inhibiting Escherichia coli and Staphylococcus aureus with a bacteriostatic rate of 90% or more.

Hydrogen peroxide has been used in tooth whitening for 40 years, and its effect and safety are higher than other tooth whitening ingredients. At present, hydrogen peroxide is still the commonly used or auxiliary active ingredient in household tooth whitening products and professional tooth whitening manners. The traditional whitening tooth paste has the following problems: (1) hydrogen peroxide will oxidize the adhesive in the product formula, resulting in the collapse of the gel skeleton structure and poor stability of the product over time; (2) a gel layer of the whitening tooth paste has poor water resistance, resulting in a short time for hydrogen peroxide to act on teeth, and therefore the whitening effect is not obvious, which cannot meet the user's requirements; (3) hydrogen peroxide will bring many side effects to teeth and oral cavity, such as damage to mechanical hardness of enamel surface, irritation to soft tissue, tooth sensitivity, acute pulpitis and other problems.

In addition, some exogenous or endogenous factors often cause teeth to show colored spots or plaque appearance, which seriously affects the beauty of teeth. Cold-light whitening technology can remove pigments by a whitening agent, such as carbamide peroxide, hydrogen peroxide or hypochlorous acid, which generates free radicals to perform a redox reaction with pigment molecules on the surface and in deep layer of teeth. However, the use of whitening agent will lead to different degrees of tooth sensitivity, and is prone to secondary staining, which will damage people's health after long-term use.

Remineralization therapy is one of the common methods for the treatment of dental caries, which means using artificial methods to re-mineralize the demineralized enamel or cementum, restoring its hardness and terminating or eliminating early caries. Remineralization therapy is mainly directed to susceptible persons for preventing early enamel caries (chalky spot or brown spot) and caries of smooth tooth surfaces. The remineralization solution used in the remineralization therapy mainly contains calcium, phosphorus and fluorine in different proportions. The addition of fluorine may significantly promote the remineralization of enamel. The pH of the remineralization solution is generally adjusted to 7. The acidic environment may weaken the mineralization effect of the mineralization solution. The application method of remineralization therapy includes the following steps: (1) preparing into a gargle for gargling daily; and (2) local application: cleaning and drying the tooth surface, and placing a cotton ball soaked with mineralization solution on the affected part for a few minutes each time, and repeating for 3-4 times.

The advantages of remineralization mechanism may make up for the shortcomings of traditional whitening tooth strip and cold-light whitening technology. At the same time, the remineralization solution used in remineralization treatment has certain remineralization ability, but it is inconvenient to use, and its remineralization efficacy needs to be improved. Therefore, it is of great significance to develop tooth gel and tooth strip with remineralization ability so as to prevent or repair demineralized tooth tissue conveniently and effectively, and, for the whitening tooth strip, to improve the safety and reliability of tooth strip whitening technology.

### SUMMARY

In order to realize minimally-invasive tooth whitening, prevent oral diseases such as caries and periodontal diseases, improve the safety of the tooth whitening technology, enhance the remineralization treatment effect of dental diseases such as shallow caries and dentin hypersensitivity, and facilitate the use of remineralization products, the present application provides a tooth strip and a tooth gel with remineralization capability and a preparation method of the tooth gel. The tooth gel and tooth strip are functional tooth gel and tooth strip that contain bioactive ingredients (including bioactive glass and calcium phosphate), respectively.

The purpose of the present application is achieved by the following technical solutions.

The present application firstly provides a tooth gel with remineralization capability, including the following raw material components by mass:
1-35% of active ingredient,
20-60% of thickening agent,
20-70% of solvent,
5-25% of solid dispersant,
0.1-0.8% of essence,
0. 1-1.0% of sweetener,
0.1-1.0% of pH regulator;
the raw material for preparing the active ingredient includes calcium phosphate, bioactive glass, or a combination thereof in any proportion.

Further, the raw material components also include, by mass, 0.1-1% of stabilizer.

The present application further provides a tooth gel with remineralization capability, including the following raw material components by mass:
1-35% of active ingredient,
20-60% of thickening agent,
20-70% of solvent,
5-25% of solid dispersant,
0.1-0.8% of essence,
0.1-1% of stabilizer,
0.1-1.0% of pH regulator;
the raw material for preparing the active ingredient includes calcium phosphate, bioactive glass, or a combination thereof in any proportion.

Further, the raw material components also include, by mass, 0.1-1.0% of sweetener.

Further, the calcium phosphate is amorphous calcium phosphate (ACP), casein phosphopeptide-calcium phosphate complex (ACP-CPP), calcium hydrogen phosphate dihydrate (Brushite), octacalcium phosphate (OCP), β-tricalcium phosphate (β-TCP), an apatite-like phase ((Ca/Sr/Mg)₁₀(PO₄)₆/((OH/F/Cl)₂/CO₃)), or any combination thereof in any proportion; where the apatite-like phase is one or more of hydroxyapatite (Ca₁₀(PO₄)₆(OH)₂), fluorapatite (Ca₁₀(PO₄)₆F₂), chlorapatite (Ca₁₀(PO₄)₆Cl₂) and composite apatite (X₁₀(PO₄)₆Y₂, where X is selected from at least one of Ca, Sr and Mg, and Y is selected from at least one of OH, F, Cl and CO₃).

Further, the bioactive glass is amorphous bioactive glass or bioactive glass-ceramic containing a crystalline phase in its bioactive glass matrix. The raw material components of the bioactive glass include: SiO₂, P₂O₅, and one or more selected from Li₂O, Na₂O, CaO, SrO, MgO, SnO, K₂O, CaF₂, SrF₂, SrCl₂, NaF, LaF₃, KF, CaCl₂, LaCl₃, KCl, MgCl₂, CuO and ZnO, and a content of P₂O₅ in the components of the bioactive glass is more than 2.0mol%; when metal fluoride is included, a total content of the metal fluoride in the components of the bioactive glass is less than 9.3mol%; the contents of the raw material components of the bioactive glass enable a network connectivity NC of the bioactive glass to meet the following: 1.5≤NC≤3.2, where a calculation formula of NC is NC=[4× M_{SiO₂} -2×(M_{MO}+M_{N₂O})+6×M_{P₂O₅}]/M_{SiO₂}, and in the formula: M_{SiO₂} is a molar percentage content of SiO₂ in a medical bioactive glass; M_{MO} is a molar percentage content of alkaline earth metal oxide in the medical bioactive glass, M_{N₂O} is a molar percentage content of alkali metal oxide in the medical bioactive glass, and M_{P₂O₅} is a molar percentage content of P₂O₅ in the medical bioactive glass; and the bioactive glass is prepared by a high-temperature melting and cold-quenching method.

Further, the raw material components of the bioactive glass include: SiO₂, P₂O₅, and one or more selected from Li₂O, CaO, SrO, MgO, SnO, K₂O, CaF₂, SrF₂, SrCl₂, NaF, LaF₃, KF, CaCl₂, LaCl₃, KCl, MgCl₂, CuO and ZnO, and a content of P₂O₅ in the components of the bioactive glass is more than 2.0mol%;
when metal fluoride is included, a total content of the metal fluoride in the components of the bioactive glass is less than 9.3mol%;
the contents of the raw material components of the bioactive glass enable a network connectivity NC of the bioactive glass to meet the following: 1.5≤NC≤3.2,
where a calculation formula of NC is NC=[4× M_{SiO₂}-2×(M_{MO}+M_{N₂O})+6× M_{P₂O₅}]/M_{SiO₂}, and in the formula: M_{SiO₂} is a molar percentage content of SiO₂ in the medical bioactive glass; M_{MO} is a molar percentage content of alkaline earth metal oxide in the medical bioactive glass, M_{N₂O} is a molar percentage content of alkali metal oxide in the medical bioactive glass, and M_{P₂O₅} is a molar percentage content of P₂O₅ in the medical bioactive glass; and
the bioactive glass is prepared by a high-temperature melting cold-quenching method.

More preferably, when the raw material components of the bioactive glass include at least one of functional metal oxides SnO, CuO and ZnO, a content of each functional metal oxide in the components of the bioactive glass is less than 5.0mol%; and a content of P2O5 in the components of the bioactive glass is more than 3.5mol%; when the metal fluoride is included, a total content of the metal fluoride in the components of the bioactive glass is less than 6.0mol%; the contents of the raw material components of the bioactive glass meet the following: 1.5≤NC≤2.4.

Further, the raw material for preparing the active ingredients also includes one or more of desensitizer, whitening agent, antibacterial agent, and anti-caries ingredient.

Further, the desensitizer is potassium nitrate, oxalic acid, calcium oxalate, tea polyphenol, catechol, glyceryl linoleate, glyceryl oleate, or any combination thereof in any proportion; the whitening agent is carbamide peroxide, hydrogen peroxide, sodium hypochlorite, or any combination thereof in any proportion; the antibacterial agent is selected from one of a compound or complex capable of releasing silver ions, copper ions, strontium ions, cobalt ions and zinc ions, or from any combination thereof in any proportion; the anti-caries component is selected from one of fluorine-containing bioactive glass that is capable of releasing fluoride ions and soluble fluoride, or from any combination thereof in any proportion. The soluble fluoride may be selected from at least one of sodium fluoride, potassium fluoride, fluorine-containing ammonium salt, sodium monofluorophosphate, lanthanum fluoride, etc.

Further, the thickening agent is selected from one of sodium polyacrylate, cellulose, N-vinyl amide polymer, carbomer, sodium alginate, gelatin and the like, or from any combination thereof in any proportion; the solvent is selected from one of glycerol, absolute ethanol and water, or from any combination thereof in any proportion; the solid dispersant is selected from polyethylene glycol 400; the sweetener is selected from acesulfame potassium; the essence is selected from essence such as menthol; the pH regulator is selected from one of NaOH, KOH, NaHCO3 or H3PO4.

The tooth gel of the present application may be used as a separate commodity for oral tooth desensitization, whitening, antibiosis, caries prevention and the like, and may also be used for oral tooth desensitization, whitening, caries prevention and the like in combination with a tooth strip, a denture, etc.

The present application also provides a tooth strip with remineralization capability, including a three-layer structure: an anti-sticking liner, a dental gel layer, and a peel-off backing layer, where the anti-sticking liner is adhered to one side of the dental gel layer, and the other side of the dental gel layer is adhered to the peel-off backing layer. The dental gel layer is the tooth gel with remineralization capability with a thickness of 100-300 um. The anti-sticking liner is selected from one of a polymer, a fabric or a film, one side of the anti-sticking liner is attached with a dental gel layer, and the other side of the anti-sticking liner is provided with an anti-sticking treatment to prevent lips from being adhered. The peel-off backing layer is selected from one of a PET sheet, a foil, a plastic sheet or a paper.

The present application further provides a method for preparing the above-mentioned tooth gel with remineralization capability, including the following steps:
1) accurately weighing each kind of raw materials according to a formula;
2) adding a thickening agent, a solid dispersant and an active ingredient into a solvent for homogeneous emulsification to obtain uniformly dispersed and completely mixed and dissolved materials;
3) keeping the materials obtained in the step 2) at a temperature of 60°C, adding a stabilizer, an essence and a sweetener, and uniformly stirring;
4) slowly adding a pH regulator into the materials obtained in the step 3), and uniformly stirring; and
5) allowing the materials obtained in the step 4) to stand for 24 hours, thereby obtaining the tooth gel with remineralization capability.

In the preparation method of the present application, the uniformly stirring in step 3) means setting a rotation speed of a stirrer to be 25-35 rpm and stirring for 10 minutes.

In the step 4), when a temperature of the material obtained in the step 3) is reduced to 40-45 °C, a whitening agent is added to prepare a whitening tooth gel or tooth strip.

The uniformly stirring in the step 4) means setting a rotation speed of the stirrer to be 15-25 rpm, and stirring until a resulting mixture is uniform.

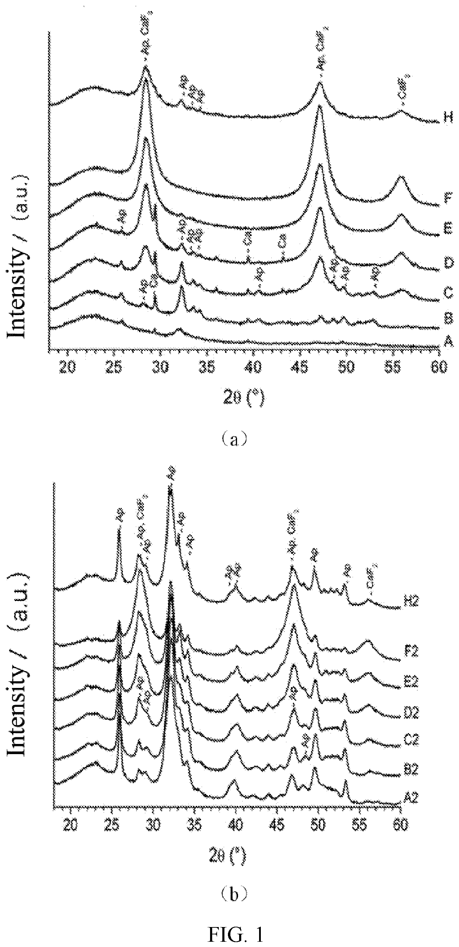

Compared with the prior art, the present application has the following advantages:
1. In the prior art, the glass of the whitening tooth strip containing a bioactive glass has low bioactivity (for example, Chinese Patent Application No.201910763871.1 provides a whitening tooth strip containing a photo-catalyst and a preparation process thereof, where a glass component has NC of 2; and P₂O₅ has a low content (1.29mol%), while CaF₂ has a very high content (9.38mol%)); while the tooth gel or tooth strip with remineralization capability described in the present application takes bioactive glass or calcium phosphate as an active ingredient, and the bioactive glass involved is a kind of bioactive glass that is personalized according to application requirements. Meanwhile, P₂O₅ in the bioactive glass of the present application has a content more than 2mol%, because the inventor has found that increasing a content of P₂O₅ in the bioactive glass may increase the rate and yield of apatite generated from the bioactive glass, thereby improving the remineralization capacity of the material (as shown in FIG. 1). In addition, the inventor also found that when the bioactive glass contains fluoride, such as CaF₂, SrF₂, LaF₃ or a mixture thereof, and more importantly, when a content of the fluoride in the bioactive glass is less than 9.3mol%, the formation of acid-resistant fluorapatite rather than CaF₂ or SrF₂ is promoted, thereby improving the remineralization capacity of the material.
2. The tooth gel and the tooth strip with remineralization capability described in the present application are convenient to carry and use; have the functions of desensitization, remineralization, antibiosis, caries prevention, whitening and the like; have little or no irritation to oral soft tissues; and have important effects and significance for preventing and treating oral diseases and improving people's health.

### BRIEF DESCRIPTION OF DRAWINGS

These and/or other aspects and advantages of the present application will become more apparent and more readily appreciated from the following detailed description of embodiments of the present application taken in conjunction with the accompanying drawings, in which:
FIG. 1 is X-ray diffraction patterns of the products obtained after soaking a bioactive glass containing different contents of phosphorus in a buffer solution for one week, of which figure (a) belongs to low-phosphorus bioactive glass, and figure (b) belongs to high-phosphorus bioactive glass.
FIG. 2 is a scanning electron micrograph of the distribution of bioactive glass in a tooth strip with remineralization capability obtained in Example 1 of the present application.
FIG. 3 is a diagram showing the whitening efficacy of a tooth strip with remineralization capability obtained in Example 1 of the present application on in vitro model of stained-teeth caused by different substances, after 3 days, 7 days and 14 days of use, in which the substance in figure (a) is coffee, and the substance in figure (b) is black tea.
FIG. 4 is a diagram showing the whitening efficacy of the tooth strip with remineralization capability obtained in Example 2 of the present application on in vitro model of stained-teeth caused by (a) coffee and (b) black tea, after 3 days, 7 days and 14 days of use.
FIG. 5 is a diagram showing that the tooth strips obtained in Examples and Comparative Examples of the present application have equally good whitening efficacy after use.
FIG. 6 is a diagram showing the formation of a remineralization layer on the enamel surface of the in vitro teeth after 7 days of treatment with each of the tooth strips obtained in Example 1 and Comparative Example 1 of the present application.
FIG. 7 is a graph comparing the effects of the tooth strips of Examples and Comparative Example of the present application on coffee-stained enamel hardness.
FIG. 8 is a diagram showing the repair-remineralization capability of the tooth strips obtained in Example 3 and Comparative Example 1 of the present application.
FIG. 9 is a structural diagram of the tooth strip with remineralization capability obtained in Example 1 of the present application (in the figure: 1. peel-off backing layer; 2. dental gel layer; 3. anti-sticking liner).

### DESCRIPTION OF EMBODIMENTS

The present application is further described in detail through embodiments, however, those embodiments should not be considered as a limitation of the present application.

### Example 1

A tooth strip with remineralization capability was prepared by a three-layer structure: an anti-sticking liner, a dental gel layer, and a peel-off backing layer, where the anti-sticking liner was tightly adhered to one side of the dental gel layer, the dental gel layer had a thickness of 100um, active ingredients were dispersed in the dental gel layer, and the other side of the dental gel layer was tightly adhered to the peel-off backing layer.

The anti-sticking liner was a transparent soft film adhesive attachment layer and was selected from natural polymers.

The peel-off backing layer was a transparent film and was a sheet selected from PET material.

The dental gel layer was a water-insoluble film layer and was prepared from the following components in percentage by mass:
35% of active ingredient;
20% of thickening agent;
25% of solvent;
19% of solid dispersant;
0.2% of essence;
0.3% of sweetener;
0.5% of pH regulator.

The active ingredient was prepared from a bioactive ingredient, a desensitizer, a whitening agent, an antibacterial agent and an anti-caries ingredient, with a mass ratio of 6:1:1:1:1.

The bioactive ingredient was a bioactive glass.

The bioactive glass was a bioactive glass-ceramic containing a small amount of crystalline phase in its matrix, and is prepared from SiO₂, P₂O₅, CaF₂, CaO and Na₂O by a high-temperature melting cold-quenching (melt-quench) method, where P₂O₅ had a content of 5.5mol%; a fluoride CaF₂ was included in a content of 6.0mol%; in addition, the contents of the components of the bioactive glass satisfied: NC is 2.0.

The desensitizer was an equal-mass mixture of potassium nitrate and glyceryl oleate.

The whitening agent was an equal-mass mixture of carbamide peroxide, hydrogen peroxide and hypochlorous acid.

The antibacterial agent was a mixture of compounds that can release silver ions and copper ions respectively, in a mass ratio of 1:2.

The anti-caries ingredient was a mixture of sodium fluoride, potassium fluoride, fluoride-containing ammonium salt, sodium monofluorophosphate and lanthanum fluoride in any proportion.

The thickening agent was selected from sodium polyacrylate.

The solvent was selected from a mixture of glycerol and absolute ethanol in equal volume.

The solid dispersant was selected from polyethylene glycol 400.

The sweetener was selected from acesulfame potassium.

The pH regulator was selected from NaOH.

A method for preparing the above mentioned tooth strip with remineralization capability included the following steps:
1) accurately weighing each kind of raw materials according to a proportion of components of the dental gel layer;
2) adding the weighed thickening agent, solid dispersant and active ingredients into a solvent, performing homogeneous emulsification in a stirrer until these components are uniformly dispersed, completely mixed and dissolved;
3) keeping the materials in the step 2) at a temperature of 60°C, adding a stabilizer and an essence, setting a rotation speed of the stirrer to be 30 rpm, and stirring for 10 minutes;
4) preparing a whitening tooth strip: adding a whitening agent when the temperature of the materials in the step 3) is reduced to 40-45°C;
5) slowly adding a pH regulator into the above-mentioned materials, and simultaneously starting the stirrer and stirring at a rotation speed of 20 rpm until the materials are uniformly mixed;
6) stand for 24 hours, then inspecting, coating the qualified mixture uniformly on a plastic film, and laminating;
7) machine-cutting and molding the laminated semi-finished product according to the shape of the tooth strip, and packaging.

The use method of the tooth strip included tearing the tooth strip off from the peel-off backing layer, sticking one side of the dental gel layer on a buccal side of a dental crown, placing a long edge of the dental gel layer to be flush with a gingival margin, and gently pressing the tooth strip to make it fully adhered to the teeth for 30-60 minutes, tearing off the tooth strip, and gargling properly to clean the teeth as required.

The formula of the dental gel layer in this Example can be separately used or packaged as a tooth gel product. It was a gel-like gel, which was used in combination with a thermoset resin denture sheet, that was to say, the thermoset resin denture sheet was placed in hot water of 70-80°C for 10-30 seconds to be softened and then taken out, and the softened denture sheet was sleeved on the teeth and pressed and shaped by a finger and removed; a proper amount of the tooth gel was introduced into the removed shaped denture sheet; and then the denture was put on and kept for 15-30 minutes and removed, and mouth was rinsed properly to clean the teeth as required. During use of the gel, it was not allowed for eating, drinking and smoking.

Similarly, the dental gel layer formula in other Examples can also be prepared according to the composition and contents of the formula to obtain a separate tooth gel product, which can be packaged as a commodity, or used in combination with an orthodontic denture, or used separately for minimally invasive tooth whitening, prevention of dental caries and prevention of periodontal disease.

### Example 2

A tooth strip with remineralization capability, which was prepared by a three-layer structure: an anti-sticking liner, a dental gel layer, and a peel-off backing layer, where the anti-sticking liner was tightly adhered to one side of the dental gel layer, the dental gel layer had a thickness of 300um, active ingredients were dispersed in the dental gel layer, and the other side of the dental gel layer was tightly adhered to the peel-off backing layer.

The anti-sticking liner was an opaque soft film adhesive attachment layer and was selected from fabric.

The peel-off backing layer was a transparent film and was selected from a foil of PET material.

The dental gel layer was a water-insoluble film layer and was prepared from the following components in percentage by mass:
20% of active ingredient;
41% of thickening agent;
20% of solvent;
17.8% of solid dispersant;
0.1% of essence;
1.0% of sweetener;
0.1% of pH regulator.

The active ingredient was prepared from a bioactive ingredient, a whitening agent, an antibacterial agent and an anti-caries ingredient, with a mass ratio of 6:1:1:2;

The bioactive ingredient was a bioactive glass.

The bioactive glass was an amorphous bioactive glass, and prepared from SiO₂, P₂O₅, Li₂O, SrO, MgO, SnO, SrF₂, SrCl₂ and KCl by a high-temperature melting cold-quenching (melt-quench) method, where P₂O₅ had a content of 4.0mol%; a fluoride SrF₂ was included in a content of 9.0mol%; in addition, the contents of the components of the bioactive glass satisfied: NC is 2.4.

The whitening agent was hydrogen peroxide.

The antibacterial agent was a mixture of compounds that can release silver ions, copper ions and strontium ion respectively, in a mass ratio of 1:2:1.

The anti-caries ingredient was sodium fluoride.

The thickening agent was selected from cellulose.

The solvent was selected from a mixture of glycerol and water in a volume ratio of 1:2.

The solid dispersant was selected from polyethylene glycol 400.

The sweetener was selected from acesulfame potassium.

The pH regulator was selected from KOH.

A method for preparing the above mentioned tooth strip with remineralization capability included the following steps:
1) accurately weighing each kind of raw materials according to a proportion of components of the dental gel layer;
2) adding the weighed thickening agent, solid dispersant and active ingredients into a solvent, performing homogeneous emulsification in a stirrer until these components are uniformly dispersed, completely mixed and dissolved;
3) keeping the materials in the step 2) at a temperature of 60°C, adding a stabilizer and an essence, setting a rotation speed of the stirrer to be 30 rpm, and stirring for 10 minutes;
4) preparing a whitening tooth strip: adding a whitening agent when the temperature of the materials in the step 3) is reduced to 40-45°C;
5) slowly adding a pH regulator into the above-mentioned materials, and simultaneously starting the stirrer and stirring at a rotation speed of 20 rpm until the materials are uniformly mixed;
6) stand for 24 hours, then inspecting, coating the qualified mixture uniformly on a plastic film, and laminating;
7) machine-cutting and molding the laminated semi-finished product according to the shape of the tooth strip, and packaging.

The use method of the tooth strip included tearing the tooth strip off from the peel-off backing layer, sticking one side of the dental gel layer on a buccal side of a dental crown, placing a long edge of the dental gel layer to be flush with a gingival margin, and gently pressing the tooth strip to make it fully adhered to the teeth for 30-60 minutes, tearing off the tooth strip, and gargling properly to clean the teeth as required.

### Example 3

A tooth strip with remineralization capability was prepared by a three-layer structure: an anti-sticking liner, a dental gel layer, and a peel-off backing layer, where the anti-sticking liner was tightly adhered to one side of the dental gel layer, the dental gel layer had a thickness of 200um, active ingredients were dispersed in the dental gel layer, and the other side of the dental gel layer was tightly adhered to the peel-off backing layer.

The anti-sticking liner was a transparent soft film adhesive attachment layer and was selected from a film.

The peel-off backing layer was an opaque film and was selected from paper of PET material.

The dental gel layer was a water-insoluble film layer and prepared from the following components in percentage by mass:
3.1% of active ingredient;
20% of thickening agent;
70% of solvent;
5% of solid dispersant;
0.6% of essence;
1.0% of sweetener;
0.3% of pH regulator.

The active ingredient was prepared from a bioactive ingredient, an antibacterial agent and an anti-caries ingredient, with a mass ratio of 6:1:1.

The bioactive ingredient was a bioactive glass.

The bioactive glass was a bioactive glass-ceramic containing a small amount of crystalline phase in its matrix, and was prepared from SiO₂, P₂O₅, CaO, K₂O, CuO and CaCh by a high-temperature melting cold-quenching (melt-quench) method, where P₂O₅ had a content of 2.5mol%, and CuO had a content of 2.0mol%; in addition, the contents of the components of the bioactive glass satisfied: NC is 3.2.

The antibacterial agent was a mixture of compounds that can release copper ions and strontium ions respectively in a mass ratio of 1:2.

The anti-caries ingredient was lanthanum fluoride.

The thickening agent was a mixture of N-vinyl amide polymer and sodium alginate in a mass ratio of 1:1.

The solvent was selected from glycerol.

The solid dispersant was selected from polyethylene glycol 400.

The sweetener was selected from acesulfame potassium.

The pH regulator was selected from NaHCO₃.

A method for preparing the above mentioned tooth strip with remineralization capability included the following steps:
1) accurately weighing each kind of raw materials according to a proportion of components of the tooth strip;
2) adding the weighed thickening agent, solid dispersant and active ingredient into a solvent, and performing homogeneous emulsification in a stirrer until these components are uniformly dispersed, completely mixed and dissolved;
3) keeping the materials in the step 2) at a temperature of 60°C, adding a stabilizer and an essence, setting a rotation speed of the stirrer to be 30 rpm, and stirring for 10 minutes;
4) slowly adding a pH regulator into the above-mentioned materials, and simultaneously starting the stirrer and stirring at a rotation speed of 20 rpm until the materials are uniformly mixed;
5) stand for 24 hours, then inspecting, coating the qualified mixture uniformly on a plastic film, and laminating;
6) machine-cutting and molding the laminated semi-finished product according to the shape of the tooth strip, and packaging.

The use method of the tooth strip included tearing the tooth strip off from the peel-off backing layer, sticking one side of the dental gel layer on a buccal side of a dental crown, placing a long edge of the dental gel layer to be flush with a gingival margin, and gently pressing the tooth strip to make it fully adhered to the teeth for 30-60 minutes, tearing off the tooth strip, and gargling properly to clean the teeth as required.

### Example 4

A tooth gel with remineralization capability was provided.

The tooth gel was prepared from the following components in percentage by mass:
15% of active ingredient;
30% of thickening agent;
32.7% of solvent;
20% of solid dispersant;
0.8% of essence;
0.5% of sweetener;
1.0% of pH regulator.

The active ingredient was prepared from a bioactive ingredient and a desensitizer, with a mass ratio of 6:1.

The bioactive glass was a casein phosphopeptide-calcium-phosphorus complex.

The desensitizer was an equal-mass mixture of oxalic acid, calcium oxalate, catechol, and glyceryl linoleate.

The thickening agent was selected from N-vinyl amides polymer.

The solvent was an equal-volume mixture of absolute ethanol and water.

The solid dispersant was selected from polyethylene glycol 400.

The sweetener was selected from acesulfame potassium.

The pH regulator was selected from NaHCO₃.

A method for preparing the above mentioned tooth gel with remineralization capability included the following steps:
1) accurately weighing each kind of raw materials according to a proportion of components of the tooth gel;
2) adding the weighed thickening agent, solid dispersant and active ingredient into a solvent, and performing homogeneous emulsification in a stirrer until these components are uniformly dispersed, completely mixed and dissolved;
3) keeping the materials in the step 2) at a temperature of 60°C, adding a stabilizer and an essence, setting a rotation speed of the stirrer to be 30 rpm, and stirring for 10 minutes;
4) slowly adding a pH regulator into the above-mentioned materials, and simultaneously starting the stirrer and stirring at a rotation speed of 20 rpm until the materials are uniformly mixed;
5) sealing and packaging after the resulting product is inspected to be qualified.

The use method of the tooth gel included taking a proper amount of the tooth gel and evenly smearing it on the tooth surface to form a thin layer of tooth gel with a thickness of about 0.5 mm; taking a proper amount of matched anti-sticking liner film to cover an outer side of the tooth gel so as to prevent the tooth gel from being adhered to lips, keeping for 15-30 minutes; wiping or tearing off the thin layer of tooth gel, and gargling properly to clean the teeth as required. During use of the gel, it was not allowed for eating, drinking and smoking.

### Example 5.

A whitening tooth gel with remineralization capability was provided.

The tooth gel was prepared from the following components in percentage by mass:
1% of active ingredient;,
60% of thickening agent;
26.7% of solvent;
10% of solid dispersant;
0.8% of essence;
0.5% of sweetener;
1.0% of pH regulator.

The active ingredient was a bioactive ingredient.

The bioactive ingredient was a mixture of amorphous calcium phosphate and a bioactive glass, with a mass ratio of 1:1.

The bioactive glass was a bioactive glass-ceramic containing a small amount of crystalline phase in its matrix, and was prepared from SiO₂, P₂O₅, CaO, CaF₂, K₂O, ZnO and CaCh by a high-temperature melting cold-quenching (melt-quench) method, where P₂O₅ had a content of 2.5mol%; a fluoride CaF₂ was included in a content of 3.0mol%; ZnO had a content of 4mol%; in addition, the contents of the components of the bioactive glass satisfied: NC is 1.5.

The thickening agent was a mixture of carbomer and gelatin in a mass ratio of 2: 1.

The solvent was an equal-volume mixture of absolute ethanol and water.

The solid dispersant was selected from polyethylene glycol 400.

The sweetener was selected from acesulfame potassium.

The pH regulator was selected from NaHCO₃.

A method for preparing the above mentioned whitening tooth gel with remineralization capability included the following steps:
1) accurately weighing each kind of raw materials according to a proportion of components of the tooth gel;
2) adding the weighed thickening agent, solid dispersant and active ingredient into a solvent, and performing homogeneous emulsification in a stirrer until these components are uniformly dispersed, completely mixed and dissolved;
3) keeping the materials in the step 2) at a temperature of 60°C, adding a stabilizer and an essence, setting a rotation speed of the stirrer to be 30 rpm, and stirring for 10 minutes;
4) preparing a whitening tooth gel: adding a whitening agent when the temperature of the materials in the step 3) is reduced to 45°C;
5) slowly adding a pH regulator into the above-mentioned materials, and simultaneously starting the stirrer and stirring at a rotation speed of 20 rpm until the materials are uniformly mixed;
6) sealing and packaging after the resulting product is inspected to be qualified.

### Comparative Example 1

The difference between this Comparative Example from Example 1 was that in this example, no bioactive glass was contained, and other components and preparation process were the same as those in Example 1.

### Comparative Example 2

Compared with Example 1, the bioactive glass in the tooth strip formula of Comparative Example 2 was the bioactive glass involved in patent CN201910763871.1 (that is, it was composed of SiO₂, CaO, K₂O, P₂O₅, CaF₂ and Na₂O at a weight ratio of 42:21:14:3:12:15), in which a content of PzOs was low (1.29mol%) and a content of CaF₂ was high (9.38mol%). A content of P₂O₅ in the bioactive glass involved in Example 1 was greater than 2mol%, and was greater than the content involved in Comparative Example 1; while a content of CaF₂ was 6.0mol%. The other relevant conditions of Example 1 are the same with those of Comparative Example 1.

Studies have shown that increasing a content of P₂O₅ in the bioactive glass can increase the rate and yield of apatite formation from the bioactive glass, thereby improving the remineralization capacity of the material (as shown in FIG. 1). In addition, the bioactive glass involved in Example 1 contains CaF₂ with a content of 6.0mol%, which leads to generation of acid-resistant fluorapatite rather than calcium fluoride crystals after glass degradation, thereby providing better remineralization capacity of material.

### Experimental Examples

FIG. 1 shows X-ray diffraction patterns of products of two series of bioactive glass with different phosphorus contents, the product being obtained after soaking the bioactive glass in a buffer solution for one week, in which FIG. 1(a) belongs to low-phosphorus bioactive glass (glass number is A-H, and a phosphorus content of the glass is about 1mol%), FIG. 1(b) belongs to high-phosphorus bioactive glass (glass number is A2-H2, and its phosphorus content is about 5mol%), and the main difference between two pieces of glass with the same letter number lies in phosphorus content.

Taking the glass A in FIG. 1(a) and the glass A2 in FIG. 1(b) as examples, the inventors found that X-ray diffraction peaks matched with apatite can be seen in the XRD patterns of both the low-phosphorus bioactive glass (A) and the high-phosphorus bioactive glass (A2). However, the intensity of the X-ray diffraction peak of apatite generated in the glass A2 is much larger than that in the glass A, revealing that the high-phosphorus bioactive glass can produce apatite faster and more than the low-phosphorus bioactive glass.

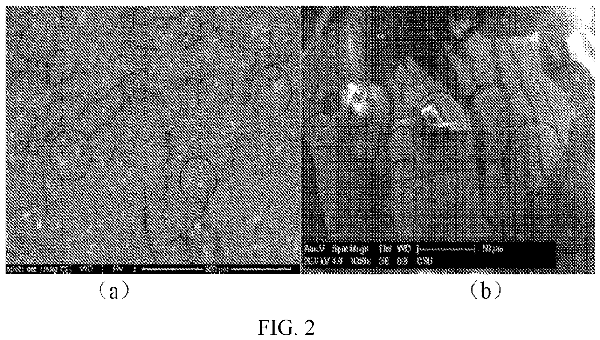

FIG. 2 shows scanning electron micrographs of the distribution of bioactive glass in the tooth strip with remineralization capability obtained in Example 1 and Example 2 of the present application, in which, FIG. (a) shows the distribution of bioactive glass in the tooth strip obtained in Example 1; and FIG. (b) shows the distribution of bioactive glass in the tooth strip obtained in Example 2.

As shown in FIG. 2(a), the bioactive glass was uniformly dispersed in the tooth strip obtained in Example 1 of the present application. As shown in FIG. 2(b), the bioactive ingredient was uniformly dispersed in the tooth strip obtained in Example 2 of the present application. The small white particles in the figures are all bioactive ingredients (as indicated by circles).

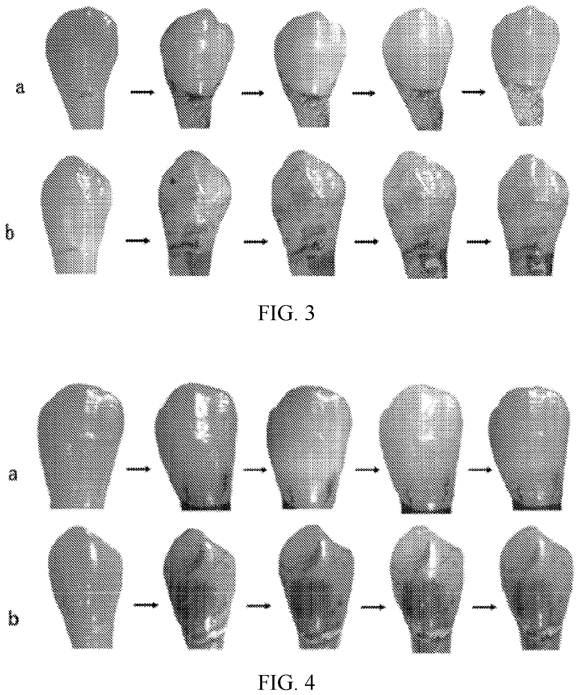

FIG. 3 is a diagram showing the whitening efficacy of a tooth strip with remineralization capability on an in-vitro model of teeth staining caused by different substances, the tooth strip being obtained in Example 1 of the present application and used for 3, 7, and 14 days (The substance in Fig. a is coffee, from left to right: untreated - after 14 days of coffee staining - after 3 days of using the tooth strip - after 7 days of using the tooth strip - after 14 days of using the tooth strip; the substance in Fig. b is black tea, from left to right: untreated - after 14 days of black tea staining - after 3 days of using the tooth strip - after 7 days of using the tooth strip - after 14 days of using the tooth strip).

As shown in FIG. 3, the color of the in-vitro teeth becomes darker after 14 days of staining with coffee and black tea respectively. After stained in-vitro teeth treatment of 3 days, 7 days and 14 days with the tooth strip with remineralization capability obtained in Example 1, the color of the in-vitro teeth in both groups gradually becomes lighter. It can be seen that the tooth strip with remineralization capability obtained in Example 1 has a better whitening effect.

FIG. 4 is a diagram showing the whitening efficacy of a tooth strip with remineralization capability on an in-vitro model of teeth staining caused by different substances, the tooth strip being obtained in Example 2 of the present application and used for 3 days, 7 days and 14 days (The substance in Fig. a is coffee, from left to right: untreated - after 14 days of coffee staining - after 3 days of using the tooth strip - after 7 days of using the tooth strip - after 14 days of using the tooth strip; the substance in Fig. b is black tea, from left to right: untreated - after 14 days of black tea staining - after 3 days of using the tooth strip - after 7 days of using the tooth strip - after 14 day of using the tooth strip) .

As shown in FIG. 4, the color of the in-vitro teeth becomes darker after 14 days of staining with coffee and black tea. After stained in-vitro teeth treatment of 3 days, 7 days and 14 days with the tooth strip with remineralization capability obtained in Example 2, the color of the in-vitro teeth in both groups gradually becomes lighter. It can be seen that the tooth strip with remineralization capability obtained in Example 1 has a better whitening effect.

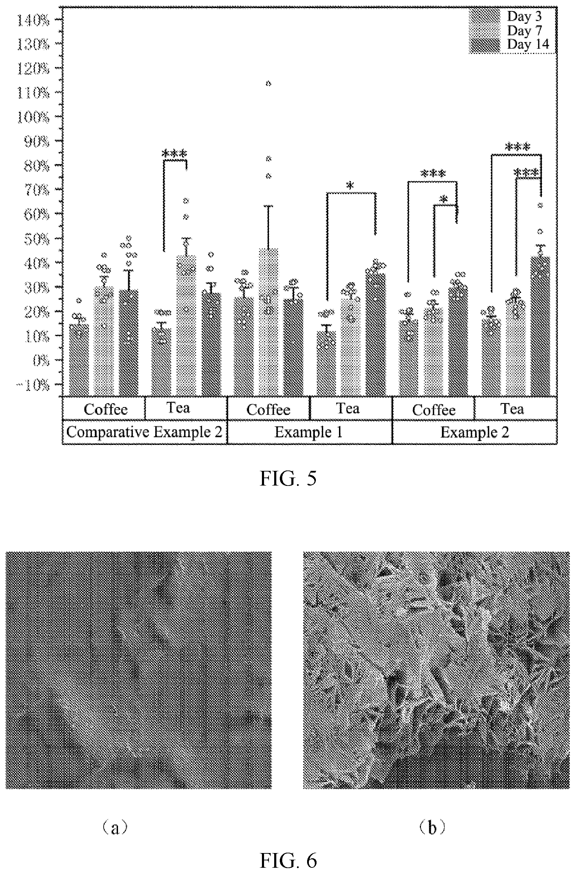

FIG. 5 is a schematic diagram of the degree of tooth whitening after use of the tooth strip obtained in Example 1, Example 2 and Comparative Example 2 of the present application (from left to right, Comparative Example 2, Example 1 and Example 2, respectively).

The tooth strips obtained in Examples 1 and 2 have a better whitening efficacy than that of Comparative Example 2 after 3 and 7 days of use. The tooth strips obtained in Examples 1 and 2 have the same good whitening efficacy as that of Comparative Example 2 after 14 days of use.

FIG. 6 is a graph showing the formation of a remineralization layer on the enamel surface of the in-vitro teeth after 7 days treatment with the tooth strips obtained in Example 1 and Comparative Example 1 of the present application respectively, in which (a) is the result of Comparative Example 1, and (b) is the result of Example 1.

As shown in FIG. 6, no obvious remineralization layer is formed on the enamel surface of the in-vitro teeth treated with the tooth strip of Comparative Example 1; and an obvious apatite remineralization layer is formed on the enamel surface of the in-vitro teeth treated with the tooth strip of Example, which has obvious repair effect on the demineralized enamel.

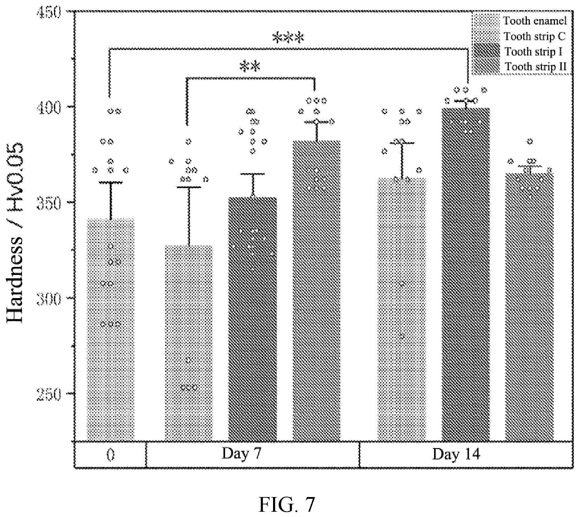

FIG. 7 is a graph comparing the effects of the tooth strips of Examples and Comparative Examples of the present application on coffee-stained enamel hardness (Comparative Example 1 is the tooth strip C in the figure, Example 1 is the tooth strip I in the figure, and Example 2 is the tooth strip II in the figure).

As shown in FIG. 7, a hardness of the tooth enamel is significantly increased after treatment with the tooth strip of Example 1 for 7 days and after treatment with the tooth strip of Example 2 for 14 days as compared to the tooth strip of Comparative Example 1.

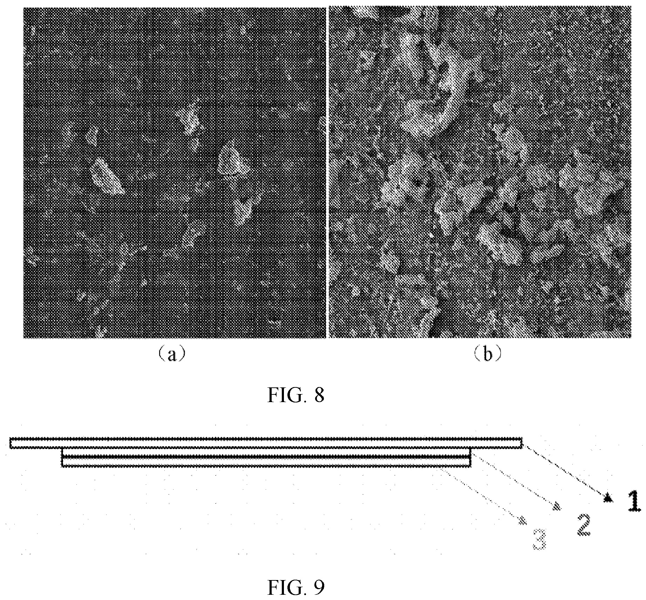

FIG. 8 is a scanning electron micrograph of the tooth strips obtained in Example 3 and Comparative Example 1 of the present application, the tooth strips being immersed in artificial saliva for 7 days, in which (a) is a scanning electron micrograph of the tooth strip obtained in Comparative Example 1 after being immersed in artificial saliva for 7 days, and (b) is a scanning electronic micrograph of the tooth strip obtained in Example 3; as shown in FIG. 8, the tooth strip of Example 3 is able to produce more apatite precipitates than Comparative Example 1 after 7 days of immersion.

FIG. 9 is a structural diagram of the tooth strip with remineralization capability obtained in Example 1 of the present application. The structure of the tooth strip with remineralization capability obtained in Example 1 of the present application includes a three-layer structure: 1) a peel-off backing layer, 2) a dental gel layer, and 3) an anti-sticking liner.

The above description is only preferred embodiment of the present application, and it should be noted that, for those of ordinary skill in the art, a number of modification and changes can be made without departing from the inventive concept of the present application, and these modification and changes fall within the scope of protection of this application.

## Claims

1. A tooth gel with remineralization capability, comprising the following raw material components by mass:
1-35% of active ingredient;
20-60% of thickening agent;
20-70% of solvent;
5-25% of solid dispersant;
0.1-0.8% of essence;
0.1-1.0% of sweetener;
0.1-1.0% of pH regulator,
the raw material for preparing the active ingredient comprises calcium phosphate, bioactive glass, or a combination thereof in any proportion.

2. The tooth gel with remineralization capability according to claim 1, wherein the raw material components further comprise, by mass, 0.1-1% of stabilizer.

3. A tooth gel with remineralization capability, comprising the following raw material components by mass:
1-35% of active ingredient;
20-60% of thickening agent;
20-70% of solvent;
5-25% of solid dispersant;
0.1-0.8% of essence;
0.1-1% of stabilizer;
0.1-1.0% of pH regulator,
the raw material for preparing the active ingredient comprises calcium phosphate, bioactive glass, or a combination thereof in any proportion.

4. The tooth gel with remineralization capability according to claim 3, wherein the raw material components further comprise, by mass, 0.1-1.0% of sweetener.

5. The tooth gel with remineralization capability according to any one of claims 1-4, wherein the calcium phosphate is amorphous calcium phosphate, casein phosphopeptide-calcium phosphate complex, brushite, octacalcium phosphate, β-tricalcium phosphate, an apatite-like phase, or any combination thereof in any proportion; wherein the apatite-like phase is one or more of hydroxyapatite, fluorapatite, chlorapatite and composite apatite.

6. The tooth gel with remineralization capability according to claim 1 or 3, wherein the raw material components of the bioactive glass comprise: SiO₂, P₂O₅, and one or more selected from Li₂O, Na₂O, CaO, SrO, MgO, SnO, K₂O, CaF₂, SrF₂, SrCl₂, NaF, LaF₃, KF, CaCl₂, LaCl₃, KCl, MgCl₂, CuO and ZnO, and a content of P₂O₅ in the raw material components of the bioactive glass is more than 2.0mol%;
when a metal fluoride is contained, a total content of the metal fluoride in the raw material components of the bioactive glass is less than 9.3mol%;
the contents of the raw material components of the bioactive glass enable a network connectivity, NC of the bioactive glass to satisfy: 1.5≤NC≤3.2, wherein NC is calculated by a formula: NC=[4× M_{SiO₂}-2×(M_{MO}+M_{N₂O})+6×M_{P₂O₅}]/M_{SiO₂}, and in the formula: M_{SiO₂} is a molar percentage content of SiO₂ in medical bioactive glass; M_{MO} is a molar percentage content of alkaline earth metal oxide in the medical bioactive glass, M_{N₂O} is a molar percentage content of alkali metal oxide in the medical bioactive glass, and M_{P₂O₅} is a molar percentage content of alkali metal oxide in the medical bioactive glass; and
the bioactive glass is prepared by a high-temperature melting-cold quenching method.

7. The tooth gel with remineralization capability according to claim 1 or 3, wherein the raw material components of the bioactive glass comprise: SiO₂, P₂O₅, and one or more selected from Li₂O, CaO, SrO, MgO, SnO, K₂O, CaF₂, SrF₂, SrCl₂, NaF, LaF₃, KF, CaCl₂, LaCl₃, KCl, MgCl₂, CuO and ZnO, and a content of P₂O₅ in the raw material components of the bioactive glass is more than 2.0mol%;
when a metal fluoride is contained, a total content of the metal fluoride in the raw material components of the bioactive glass is less than 9.3mol%;
the contents of the raw material components of the bioactive glass enable a network connectivity, NC of the bioactive glass to satisfy: 1.5≤NC≤3.2, wherein NC is calculated by a formula: NC=[4× M_{SiO₂} -2×(M_{MO}+ M_{N₂O})+6× M_{P₂O₅}]/M_{SiO₂}, and in the formula: M_{SiO₂} is a molar percentage content of SiO₂ in medical bioactive glass; M_{MO} is a molar percentage content of alkaline earth metal oxide in the medical bioactive glass, M_{N₂O} is a molar percentage content of alkali metal oxide in the medical bioactive glass, and M_{P₂O₅} is a molar percentage content of alkali metal oxide in the medical bioactive glass; and
the bioactive glass is prepared by a high-temperature melting-cold quenching method.

8. The tooth gel with remineralization capability according to claim 6 or 7, wherein when the raw material components of the bioactive glass comprise at least one of functional metal oxides SnO, CuO and ZnO, a content of each functional metal oxide in the raw material components of the bioactive glass is less than 5.0mol%; and a content of P₂O₅ in the raw material components of the bioactive glass is more than 3.5mol%;
when the metal fluoride is contained, a total content of the metal fluoride in the raw material components of the bioactive glass is less than 6.0mol%;
the contents of the raw material components of the bioactive glass satisfy: 1.5≤NC≤2.4.

9. The tooth gel with remineralization capability according to claim 1 or 3, wherein the raw material for preparing the active ingredient further comprises one or more of desensitizer, whitening agent, antibacterial agent, and anti-caries ingredient.

10. The tooth gel with remineralization capability according to claim 9, wherein the desensitizer is one of potassium nitrate, oxalic acid, calcium oxalate, tea polyphenol, catechol, glyceryl linoleate and glyceryl oleate, or any combination thereof in any proportion;
the whitening agent is one of carbamide peroxide, hydrogen peroxide and sodium hypochlorite, or any combination thereof in any proportion;
the antibacterial agent is one of compound or complexes that release silver ions, copper ions, strontium ion, cobalt ions and zinc ions, or any combination thereof in any proportion;
the anti-caries ingredient is one of fluorine-containing bioactive glass capable of releasing fluoride ions and soluble fluoride such as sodium fluoride, potassium fluoride, fluorine-containing ammonium salt, sodium monofluorophosphate, lanthanum, or any combination thereof in any proportion.

11. The tooth gel with remineralization capability according to claim 1 or 3, wherein the thickening agent is selected from one of sodium polyacrylate, cellulose, N-vinyl amide polymer, carbomer, sodium alginate, and gelatin, or from any combination thereof in any proportion;
the solvent is selected from one of glycerol, absolute ethanol and water, or from any combination thereof in any proportion;
the solid dispersant is selected from polyethylene glycol 400;
the sweetener is selected from acesulfame potassium;
the essence is selected from essence such as menthol;
the pH regulator is selected from one of NaOH, KOH, NaHCO₃ or H₃PO₄.

12. A tooth strip with remineralization capability, comprising a three-layer structure: an anti-sticking liner, a dental gel layer, and a peel-off backing layer, wherein the anti-sticking liner is adhered to one side of the dental gel layer, and the other side of the dental gel layer is adhered to the peel-off backing layer; and the dental gel layer is the tooth gel with remineralization capability according to any one of claims 1-11, and has a thickness of 100-300 um;
the anti-sticking liner is selected from one of a polymer, a fabric or a film, one side of the anti-sticking liner is attached with a dental gel layer, and the other side of the anti-sticking liner is subjected to an anti-sticking treatment to prevent lips from being adhered;
the peel-off backing layer is selected from one of a PET sheet, a foil, a plastic sheet or a paper.

13. A method for preparing the tooth gel with remineralization capability according to claim 2 or 4, comprising the following steps:
1) accurately weighing each kind of raw materials based on a formula;
2) adding a thickening agent, a solid dispersant and active ingredient into a solvent, and performing homogeneous emulsification to obtain uniformly dispersed and completely mixed and dissolved materials;
3) keeping the material obtained in the step 2) at a temperature of 60°C, adding a stabilizer, an essence and a sweetener, and uniformly stirring;
4) slowly adding a pH regulator into the materials obtained in the step 3), and uniformly stirring; and
5) allowing the materials obtained in the step 4) to stand for 24 hours to obtain the tooth gel with remineralization capability.

14. The preparation method of the tooth gel with remineralization ability according to claim 13, wherein the uniformly stirring in step 3) comprises setting a rotation speed of a stirrer to 25-35 rpm and stirring for 10 minutes;
in step 4), when a temperature of the materials obtained in the step 3) is reduced to 40-45 °C, adding a whitening agent to prepare a whitening tooth gel or a tooth strip;
the uniformly stirring in step 4) comprises setting a rotation speed of a stirrer to be 15-25 rpm, and stirring to obtain a uniform mixture.
